# FASCICULE DE BREVET EUROPEEN

(11) **EP 1 585 722 B1**
(45) Date de publication et mention de la délivrance du brevet: **09.07.2008**
(21) Numéro de dépôt: 03815395.3
(22) Date de dépôt: 12.12.2003
(51) Int. Cl.: C07C 253/10

(54) **PROCEDE DE SYNTHESE DE COMPOSES COMPRENANT DES FONCTIONS NITRILES A PARTIR DE COMPOSES A INSATURATIONS ETHYLENIQUES**
VERFAHREN ZUR SYNTHESE VON NITRILFUNKTIONEN ENTHALTENDEN VERBINDUNGEN AUS ETHYLENISCH UNGESÄTTIGTENVERBINDUNGEN
METHOD FOR SYNTHESIS OF COMPOUNDS COMPRISING NITRILE FUNCTIONS FROM ETHYLENICALLY UNSATURATED COMPOUNDS

(30) Priorité: 23.12.2002 FR 0216550
(43) Date de publication de la demande: 19.10.2005
(73) Titulaire: RHODIA POLYAMIDE INTERMEDIATES, 69192 Saint-Fons (FR)
(72) Inventeur: ROSIER, Cécile, F-69510 Soucieu en Jarrest (FR); MARION, Philippe, F-69390 Vernaison (FR); BOURGEOIS, Damien, F-69003 Lyon (FR)
(74) Mandataire: Esson, Jean-Pierre
(86) Numéro de dépôt international: PCT/FR2003/003690
(87) Numéro de publication internationale: WO 2004/065352

(56) Documents cités:
- WO-A-00/37431
- WO-A-99/06356
- US-A- 3 496 215

## Description

La présente invention concerne un procédé d'hydrocyanation de composés organiques à insaturations éthyléniques en composés comprenant au moins une fonction nitrile.

Elle se rapporte plus particulièrement à l'hydrocyanation de dioléfines telles que le butadiène ou d'oléfines substituées telles que des alcènenitriles comme les pentènenitriles.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'un phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux travaux ont été effectués pour trouver des systèmes catalytiques comprenant généralement un ligand organophosphoré et un métal catalytiquement actif, plus particulièrement le nickel et présentant des performances de plus en plus élevées.

La performance d'un système catalytique s'évalue en déterminant plusieurs caractéristiques comme notamment la stabilité de l'activité catalytique du système, le rendement de la réaction et la sélectivité dans la synthèse de produits valorisables dans le cas présent la sélectivité en pentènenitriles linéaires ou en adiponitrile.

Ainsi, il a été proposé dans un premier temps d'utiliser comme système catalytique du nickel associé à des ligands organophosphorés comprenant un seul atome de phosphore, appelé ligands monodentates. Le composé de cette classe de produits utilisé industriellement est le tritolylphosphite (TTP). Ce système a des performances très acceptables dans la synthèse de pentènitriles par hydrocyanation du butadiène mais des performances perfectibles dans la synthèse d'adiponitrile par hydrocyanation des pentènenitriles.

Par ailleurs, ce système catalytique présente une bonne stabilité et solubilité dans le milieu réactionnel. De tels systèmes catalytiques ont été décrits dans de nombreux brevets comme, par exemple, les brevets US3496215, DE19953058, FR1529134, FR2069411, US3631191, US3766231, FR2523974.

Pour obtenir des performances catalytiques, notamment une sélectivité élevée dans l'étape d'hydrocyanation des alcènenitriles, plus particulièrement des pentènenitriles, en dinitriles, il a été proposé une nouvelle classe de ligands organophosphorés destinés à être associés notamment au nickel. Cette nouvelle classe de ligands comprend les composés organophosphorés comprenant plusieurs atomes de phosphore, appelés ci-après pluridentates. Parmi ceux-ci, les composés proposés sont généralement des composés comprenant deux atomes de phosphore et appelés ligands bidentates.

De nombreux brevets protègent de tels composés et systèmes catalytiques. On peut citer à titre d'exemple : les brevets WO9906355, WO9906356, WO9906357, WO9906358, WO9952632, WO9965506, WO9962855, US5693843, WO961182, WO9622968, US5981772, WO0136429, WO9964155, WO0213964.

La structure de ces composés est plus ou moins complexe notamment au niveau des groupements portés par les atomes de phosphore et de la structure reliant les deux atomes de phosphore entre eux.

Avec cette classe de ligands bidentates, il est possible d'obtenir un système catalytique présentant notamment une meilleure sélectivité en production de dinitriles linéaires dans le procédé d'hydrocyanation d'un alcènenitrile.

Toutefois, ces ligands bidentates de structure plus complexe sont plus difficiles à synthétiser rendant leur coût plus élevé. En conséquence, il est nécessaire et important que leur stabilité dans le milieu réactionnel soit très élevée pour permettre une utilisation dans un procédé industriel, d'un point de vue économique. De plus, compte tenu de leur structure complexe, leur solubilité dans le milieu réactionnel peut être diminuée et conduire à un abaissement de l'activité catalytique et donc du rendement total de la réaction d'hydrocyanation.

Un des buts de la présente invention est de proposer une solution permettant d'utiliser des systèmes catalytiques à base de ligands pluridentates présentant des caractéristiques notamment de sélectivité élevée en diminuant les inconvénients liés à leur instabilité et leur niveau de solubilité.

A cet effet, l'invention propose un procédé d'hydrocyanation de composés comprenant au moins une insaturation éthylénique par réaction avec du cyanure d'hydrogène, en présence d'un système catalytique comprenant un élément métallique à activité catalytique associé à des ligands organophosphorés choisis dans le groupe comprenant les organophosphonites, organophosphinites, organophosphines, organophosphites et organophosphoramidites, caractérisé en ce que le système catalytique comprend au moins deux ligands organophosphorés, un premier ligand choisi dans le groupe des composés organophosphites monodentates et un deuxième ligand choisi dans le groupe des ligands organophosphorés pluridentates.

Selon une caractéristique préférentielle de l'invention, le rapport du nombre de moles de second ligand exprimé en atome de phosphore par rapport au nombre d'atomes d'élément métallique est au moins égal à 1, de préférence compris entre 1 et 6, avantageusement compris entre 1 et 5 et très préférentiellement compris entre 1 et 4.

Selon une autre caractéristique de l'invention, le rapport entre le nombre de moles total de ligands organophosphorés exprimé en nombre d'atomes de phosphore et le nombre d'atomes d'élément métallique est compris entre 2 et 100. Toutefois, ce rapport n'est pas critique.

Par nombre de moles total de ligands organophosphorés, il faut comprendre la somme des moles de premier et deuxième ligands.

Selon encore une autre caractéristique préférentielle de l'invention, le rapport entre le nombre de moles du premier ligand et le nombre de moles du deuxième ligand est avantageusement supérieur à 0,1, et encore plus avantageusement supérieur ou égal à 0,5.

Les ligands organophosphorés convenables comme premier ligand sont choisis dans le groupe comprenant les composés organophosphites monodentates tels que les composés de formule générale suivante (I)

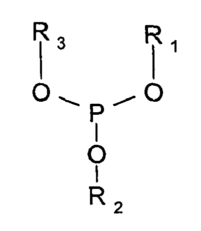

Dans laquelle
R₁, R₂, R₃ identiques ou différents représentent un radical alkyle linéaire ou ramifié ayant de 1 à 12 atomes de carbone pouvant comprendre des hétéroatomes, un radical aromatique ou cycloaliphatique substitué ou non pouvant comprendre des héréroatomes et un ou plusieurs cycles sous forme condensée ou non, les radicaux R₁, R₂, R₃ pouvant être reliés entre eux deux à deux.

A titre d'exemple de composés organophosphites monodentates convenables pour l'invention, on peut citer le triphénylphosphite, le tritolylphosphite, le trithymolphosphite.

Le tritolylphosphite est le composé préféré car il présente une bonne solubilité dans le milieu réactionnel et est d'un coût peu élevé.

Les composés organophosphorés convenables pour le deuxième ligand de l'invention sont notamment les composés organophosphorés bidentates de formule générale II suivante :

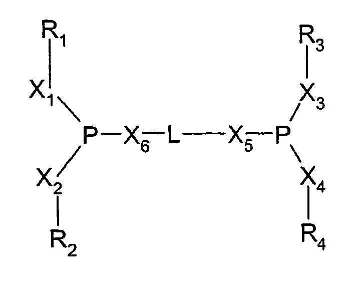

Dans laquelle,
R₁, R₂, R₃, R₄ identiques ou différent représentent un radical alkyle linéaire ou ramifié ayant de 1 à 12 atomes de carbone pouvant comprendre des hétéroatomes, un radical aromatiques ou cycloaliphatique substitué ou non pouvant comprendre des hétéroatomes et un ou plusieurs cycles sous forme condensée ou non, un radical alkylaryle, ou un radical arylalkyle, les radicaux R₁, et R₂, et/ou R₃ et R₄ pouvant être reliés entre eux
X₁, X₂, X₃, X₄, X₅, X₆ identiques ou différents représentent une liaison covalente, un atome d'oxygène ou un radical divalent - NR₅ - dans lequel R₅ représente un atome d'hydrogène ou un radical alkyle, aryle, sulfonyle cycloalkyle ou carbonylé, et
L représente une liaison covalente ou un radical divalent, alkyle linéaire ayant de 1 à 12 atomes de carbone pouvant comprendre des hétéroatomes, un radical divalent cycloaliphatique ou aromatique pouvant comprendre des hétéroatomes, substitué ou non, pouvant comprendre plusieurs cycles sous forme condensée ou non, un radical divalent alkylaryle, ou arylakyle. Comme exemples de composés organophosphorés bidentates nous pouvons citer les composés décrits dans les brevets ou demandes de brevet cités précédemment.

On peut citer également plus particulièrement comme deuxièmes ligands les composés exemplifiés dans les brevets suivants: WO 95/30680, WO 96/11182, WO 99/06358, WO 99/13983, WO 99/64155, WO 01/21579, WO 01/21580.

On donne à titre d'exemple les structures suivantes dans lesquelles ph signifie phényle :

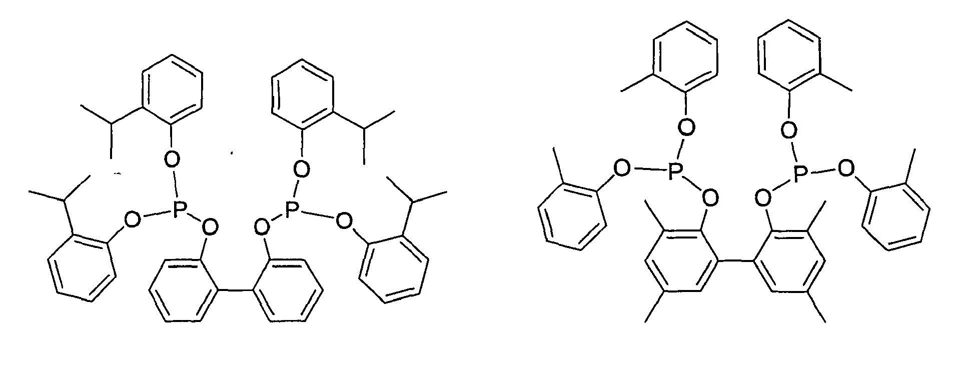

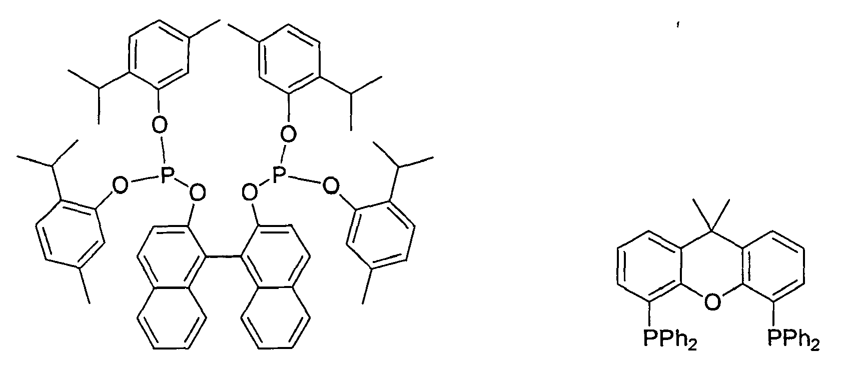

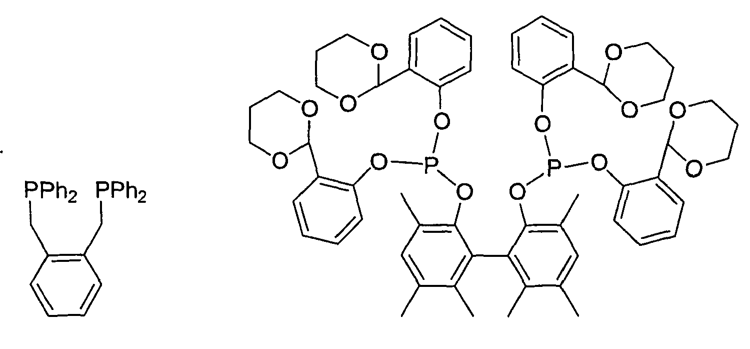

Ces composés ont une structure plus complexe que les premiers ligands décrits ci-dessus et présentent souvent une solubilité inférieure dans le milieu réactionnel et une instabilité plus importante. Toutefois, ces deuxièmes ligands ou ligands bidentates présentent de meilleures propriétés catalytiques, notamment au niveau de la sélectivité en nitriles ou dinitriles.

Selon le procédé de l'invention, l'utilisation d'un mélange deux ligands mono et pluri (bi) dentates permet de conserver les propriétés catalytiques du composé pluri (bi)dentate, notamment au niveau de la sélectivité, tout en améliorant leur stabilité dans le milieu réactionnel.

Selon l'invention, on peut symboliquement définir le système catalytique par la formule générale III suivante :

M [L₁]ₓ [L₂]_{y} (III)

Dans laquelle
M représente un métal de transition à activité catalytique.
L₁ représente le premier ligand organophosphoré monodentate
L₂ représente le deuxième ligand organophosphoré pluridentate
x; y représentent un nombre décimal correspondant au nombre de mole du ligand respectif dans le système catalytique.

Selon une caractéristique préférée de l'invention, l'élément métallique, M présentant une activité catalytique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le cérium.

Cette formule est donnée uniquement à titre indicatif et ne signifie pas que chaque atome de métal M est coordonné avec les deux ligands. Ainsi, il est possible qu'une partie des atomes du métal M soit coordonnée avec le premier ligand uniquement, l'autre partie des atomes avec le deuxième ligand et enfin une dernière partie avec les deux ligands dans des proportions variables. La formule ci-dessus n'a été donnée qu'à titre indicatif et pour une clarté de lecture et est basée sur les quantités de composés mis en oeuvre et non sur une analyse structurale du système catalytique obtenu.

La préparation des complexes organométalliques formant le système métallique ci-dessus peut être effectuée en mettant en contact une solution d'un composé du métal choisi avec une solution de chaque ligand (premier ou deuxième) ou une solution de ces deux ligands.

Le composé du métal peut être dissout dans un solvant.

Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).

Si lors de la préparation du complexe organométallique, le métal est mis en oeuvre à un degré d'oxydation plus élevé, il pourra être réduit in situ.

Comme métal de transition, les composés des métaux de transition, plus particulièrement les composés du nickel, du palladium du fer ou du cuivre sont de préférence utilisés.

Parmi les composés précités, les composés les plus préférés sont ceux du nickel.

On peut citer à titre d'exemples non limitatifs :
- les composés dans lesquels le nickel est au degré d'oxydation zéro comme le tétracyanonickelate de potassium K4 [Ni(CN)4], le bis (acrylonitrile) nickel zéro, le bis (cyclooctadiène-1,5) nickel (appelé également Ni(cod)2) et les dérivés contenant des ligands comme le tétrakis (triphényl phosphine) nickel zéro,
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH4Na, le BH4K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif. Quand on utilise un composé du fer, les mêmes réducteurs conviennent.

Dans le cas du palladium, les réducteurs peuvent être, en outre, des éléments du milieu réactionnel (composé organophosphoré, solvant, oléfine).

Les additions des solutions de premier et deuxième ligands peuvent être simultanées ou successives. En outre, il est possible de préparer séparément les complexes organométalliques avec chaque ligand puis de mélanger les deux systèmes avant de les introduire dans le milieu réactionnel, soit directement dans ledit milieu

Les composés organiques comportant au moins une double liaison éthylénique plus particulièrement mis en oeuvre dans le présent procédé sont les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

Les pentènenitriles notamment peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentènenitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition aux mélanges de premier et deuxième ligands seuls ou dissous dans un solvant, la quantité appropriée de composé du métal de transition choisi et éventuellement du réducteur ou selon les méthodes décrites ci-dessus. II est également possible de préparer le système catalytique "in situ" par simple addition des premier et deuxième ligands et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner ou isomériser comprise entre 10⁻⁴ et 1, et de préférence entre 0,0003 et 0,5 mole par mole de nickel ou d'un autre métal de transition mis en oeuvre.

La quantité totale de ligands organophosphorés utilisée pour former le catalyseur est choisie de telle sorte que le nombre de moles de ces composés rapporté à 1 mole de métal de transition soit de 0,5 à 500 et de préférence de 2 à 100.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique ou biphasique. Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu.

Le cyanure d'hydrogène est introduit dans le réacteur sous forme gazeuse ou sous forme liquide. II peut également être préalablement dissout dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le composé à hydrocyaner, les composés de formules (I) et (II), le composé de métal de transition, les éventuels réducteur et solvant, soit séparément lesdits constituants. Généralement le réacteur est alors porté à la température choisie. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés, par exemple, par distillation.

Le procédé d'hydrocyanation de composés à insaturation éthylénique selon la présente invention concerne également l'hydrocyanation desdits composés nitriles à insaturation éthylénique obtenus ci-dessus, par réaction avec le cyanure d'hydrogène et consiste à utiliser un système catalytique conforme à la présente invention avec un cocatalyseur consistant en au moins un acide de Lewis.

Les composés à insaturation éthylénique qui peuvent être mis en oeuvre dans cette étape sont de manière générale ceux qui ont été cités pour le procédé de base. Cependant il est plus particulièrement avantageux de l'appliquer à la réaction d'hydrocyanation en dinitriles, des nitriles aliphatiques à insaturation éthylénique, notamment aux pentènenitriles linéaires comme le pentène-3-nitrile, le pentène-4-nitrile et leurs mélanges.

Ces pentènenitriles peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant de la réaction antérieure d'hydrocyanation du butadiène et/ou de l'isomérisation du méthyl-2-butène-3-nitrile en pentènenitriles.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments. Ces composés sont le plus souvent des sels, notamment des halogénures, comme chlorures ou bromures, sulfates, sulfonates, halogenoalkylsulfonates, perhalogénoalkylsulfonates, notamment fluoroalkylsulfonates ou perfluoroalkylsulfonates, halogénoacétates, perhalogénoacétates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés comme le triphénylborane, l'isopropylate de titane.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane le trifluorométhylsulfonate d'indium, le trifluoroacétate d'indium, et les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel.

Comme pour la mise en oeuvre du procédé de base de l'invention, le système catalytique utilisée pour l'hydrocyanation en présence d'acide de Lewis peut être préparée avant son introduction dans la zone de réaction, ou in-situ par exemple par addition au milieu réactionnel des différents composants du système catalytique.

II est également possible dans les conditions du procédé d'hydrocyanation de la présente invention, et notamment en opérant en présence du système catalytique décrit précédemment comportant au moins un ligand monodentate et un ligand bidentate et au moins un composé d'un métal de transition, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés.

Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène, de l'adiponitrile, du méthyl-2-glutaroronitrile, de l'éthyl-2-succinonitrile ou du valéronitrile.

II est particulièrement intéressant de traiter le mélange réactionnel provenant de l'hydrocyanation du butadiène par HCN en présence d'au moins un mélange de composés de formules (I) et (II) et d'au moins un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 200°C et de préférence de 60°C à 180°C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, le système catalytique utilisé pour l'isomérisation peut être soit déjà présent dans le milieu soit préparé selon les modes de préparation déjà décrits ci-dessus.

Bien que la réaction d'isomérisation soit conduite généralement sans solvant, il peut être avantageux de rajouter un solvant organique inerte qui pourra être celui de l'extraction ultérieure. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mis en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mis en oeuvre avec un système catalytique conforme à l'invention.

Les exemples qui suivent illustrent l'invention.

Dans les exemples les abréviations utilisées ont les significations indiquées ci-dessous.
cod : 1,5-cyclooctadiène.
eq : équivalent.
3PN : 3-pentènenitrile.
4PN : 4-pentènenitrile.
3+4PN : 3PN + 4PN.
TT (Y) : taux de transformation du produit à hydrocyaner Y correspondant au rapport du nombre de moles transformées de Y sur le nombre de moles initiales de Y.
Linéarité (L) : rapport du nombre de moles d'adiponitrile (AdN) formées au nombre de moles de dinitriles formées (somme des moles de adiponitrile (AdN), éthylsuccinique dinitrile(ESN) et méthylglutaronitrile (MGN)).
Sélectivité (%) : Nombre de moles de AdN formés par rapport au nombre de moles d'AdN théorique calculé à partir du nombre de moles de 3+4pn transformées.
CPG : chromatographie phase gazeuse.
ml : millilitre.
mol : mole.
mmol : millimole.
Ph : phényle.

### Exemples 1 à 12 :

Ces essais concernent l'hydrocyanation de pentènenitriles en adiponitrile. Ces essais ont été réalisés selon quatre modes opératoires décrits ci-dessous.

### Mode opératoire n°1

Sous atmosphère inerte, dans un tube en verre type Schott de 60ml équipé d'un bouchon septum, sont chargés successivement :
- Le ligand (6 eq mole de ligand / Ni pour ligands monodentates, 3 eq mole de ligand / Ni pour ligands bidentates) ;
- Le 3-pentènenitrile (1,25 g, 400 eq / Ni) ;
- Le bis(1, 5-cyclooctadiène)₂ nickel (21mg) ;
- L'acide de Lewis (1 eq / Ni).

Le milieu réactionnel est alors chauffé sous agitation à 70°C. De la cyanhydrine de l'acétone est alimentée dans le milieu par un pousse seringue avec un débit de 0.45ml par heure.

Après 3h d'injection, l'introduction de cyanhydrine de l'acétone est arrêtée. Le mélange est refroidi à température ambiante, dilué à l'acétone et analysé par chromatographie en phase gazeuse.

### Mode opératoire 2 :

Sous atmosphère inerte, dans un tube en verre type Schott de 60ml équipé d'un bouchon septum, sont chargés successivement :
- Le ligand bidentate ( 1eq mole de ligand / Ni)
- Le ligand monodentate ( 4 eq mole de ligand/ Ni) ;
- Le 3-pentènenitrile (1,25 g, 400 eq / Ni) ;
- Le bis(1, 5-cyclooctadiène)₂ nickel (21mg) ;
- L'acide de Lewis (1 eq / Ni).

Le milieu réactionnel est alors chauffé sous agitation à 70°C. De la cyanhydrine de l'acétone est alimentée dans le milieu par un pousse seringue avec un débit de 0.45ml par heure. Après 3h d'injection, l'introduction de cyanhydrine de l'acétone est arrêtée. Le mélange est refroidi à température ambiante, dilué à l'acétone et analysé par chromatographie en phase gazeuse.

### Mode opératoire n°3 :

Sous atmosphère inerte, dans un réacteur inox de 100ml sont chargés successivement :
- Le ligand (6 eq mole de ligand/ Ni pour ligands monodentates, 3eq mole de ligand / Ni pour ligands bidentates) ;
- Le 3-pentènenitrile (30 g, 75 eq / Ni) ;
- Le bis(1, 5-cyclooctadiène)₂ nickel (1.30g);
- L'acide de Lewis (1 eq / Ni).

Le milieu réactionnel est alors chauffé sous agitation à 55°C. HCN liquide est ajouté dans le milieu réactionnel par un pousse seringue selon un débit de 1.92ml par heure. Après 5h d'injection, l'introduction de HCN est arrêtée. Le mélange est refroidi à température ambiante, dilué à l'acétone et analysé par chromatographie en phase gazeuse.

### Mode opératoire n° 4 :

Sous atmosphère inerte, un réacteur inox de 100ml sont chargés successivement :
- Le ligand bidentate ( 1eq mole de ligand / Ni )
- Le ligand monodentate ( 4 eq mole de ligand / Ni ) ;
- Le 3-pentènenitrile (30 g, 75 eq / Ni) ;
- Le bis(1, 5-cyclooctadiène)₂ nickel (1.30g);
- L'acide de Lewis (1 eq / Ni).

Le milieu réactionnel est alors chauffé sous agitation à 55°C. HCN liquide est ajouté dans le milieu réactionnel par un pousse seringue selon un débit de 1.92ml par heure. Après 5h d'injection, l'introduction de HCN est arrêtée. Le mélange est refroidi à température ambiante, dilué à l'acétone et analysé par chromatographie en phase gazeuse.

Les réactifs utilisés et les résultats obtenus sont rassemblés dans le tableau I ci-dessous :

| EX | Ligand utilisé | Rapport UNi | Acide de Lewis | Mode opératoire | L (%) | Sélectivité AdN (%) | Solubilité |
|---|---|---|---|---|---|---|---|
| 1 | Ligand C | 6 | ZnCl₂ | 1 | 86 | 79 | Oui |
| 2 | Ligand C | 2 | ZnCl₂ | 1 | 83 | 73 | Oui |
| 3 | Ligand A | 6 | ZnCl₂ | 1 | 76 | 61 | oui |
| 4 | Ligand C + A | 2-4 | ZnCl₂ | 1' | 84 | 82 | oui |
| 5 | Ligand C | 6 | ZnCl₂ | 1 | 86 | 79 | Oui |
| 6 | Ligand C | 2 | ZnCl₂ | 1 | 83 | 73 | Oui |
| 7 | Ligand B | 6 | ZnCl₂ | 1 | 54 | 13 | oui |
| 8 | Ligand C + B | 2-4 | ZnCl₂ | 1' | 83 | 74 | oui |
| 9 | Ligand D | 6 | InTFA₃ | 2 | 92 | 81 | non |
| 10 | Ligand D | 2 | InTFA₃ | 2 | 74 | (1) | oui |
| 11 | Ligand A | 6 | InTFA₃ | 2 | 81 | 75 | oui |
| 12 | Ligand D + A | 2-4 | InTFA₃ | 2' | 92 | 81 | oui |

| | | | | | | | |
|---|---|---|---|---|---|---|---|
| (1) la sélectivité n'a pu être déterminée car le taux de transformation était trop faible InTFA₃ : trifluoroacétate d'indium | | | | | | | |

Le rapport UNi est exprimé en atome de phosphore par atome de nickel, le premier rapport concerne le rapport des atomes de phosphore apportés par le second ligand, le second rapport concernant les atomes de phosphore apportés par le premier ligand
Ligand A : tritolylphosphite
Ligand B : trithymolphosphite

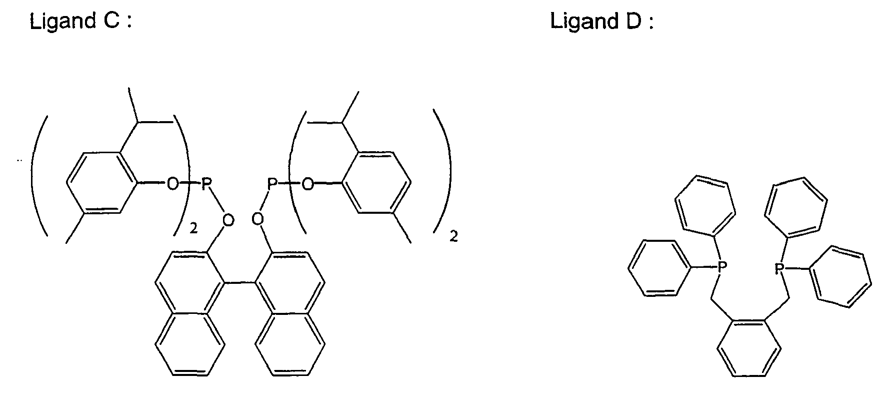

### Exemple 13 : Hydrolyse d'un mélange de ligands de formules suivantes

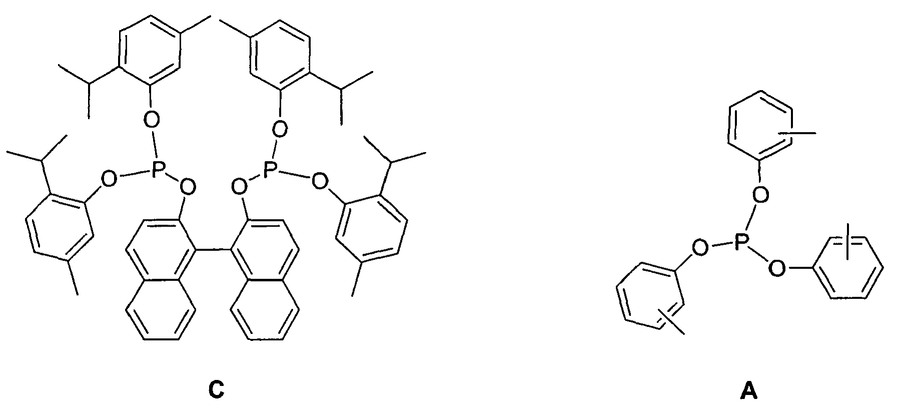

En boîte à gants on introduit dans un tube de type Schott de 30ml successivement le ligand C (270mg), le ligand A (2 éq.), du 3-pentènenitrile (1.0g), du chlorure de zinc(II) (2 éq.). On obtient un mélange clair homogène que l'on agite sous atmosphère inerte et auquel on ajoute 2 éq. d'eau. La composition du mélange est ensuite analysée par RMN ³¹P.

On obtient les résultats suivants :

| *Temps* | *Proportion en poids de C* | *Proportion en poids de A* |
|---|---|---|
| 15 min. | 35% | 60% |
| 1 h 40 | 28% | 19% |
| 4 h 30 | 28% | 14% |

Ces résultats montrent la protection contre l'hydrolyse des ligands bidentates ou deuxième ligand permettant ainsi d'économiser le ligand le plus coûteux en diminuant le taux d'hydrolyse ainsi que la quantité de ligand bidentate par rapport à l'élément métallique tout en conservant le niveau d'activité catalytique du système comprenant le deuxième ligand.

## Revendications

1. Procédé d'hydrocyanation de composés comprenant au moins une insaturation éthyléniques par réaction avec du cyanure d'hydrogène, en présence d'un système catalytique comprenant un élément métallique à activité catalytique associé à des ligands organophosphorés choisis dans le groupe comprenant les organophosphonites, organophosphinites, organophosphites organophosphines et organophosphoramidites **caractérisé en ce que** le système catalytique comprend au moins un premier ligand choisi dans le groupe des organophosphites monodentates et au moins un deuxième ligand choisi dans le groupe des ligands organophosphorés pluridentates

2. Procédé selon la revendication 1, **caractérisé en ce que** le deuxième ligand organophosphoré est un ligand bidentate.

3. Procédé selon la revendication 1 ou 2, **caractérisé en ce que** le deuxième ligand est choisi dans le groupe comprenant les composés organophosphites, organophosphines, organophosphinites, organophosphonites, organophophoramidites.

4. Procédé selon l'une des revendications précédentes, **caractérisé en ce que** le rapport entre le nombre de deuxième ligand pluridentate exprimé en atome de phosphore et le nombre d'atomes d'élément métallique est supérieur à 0,5, de préférence compris entre 1 et 5.

5. Procédé selon l'une des revendications précédentes, **caractérisé en ce que** le rapport entre le nombre total de moles de premier et deuxième ligands exprimé en nombre d'atome de phosphore et le nombre d'atomes d'élément métallique est compris entre 1 et 100.

6. Procédé selon l'une des revendications précédentes, **caractérisé en ce que** le rapport entre le nombre total de mole de premier ligand et le nombre de moles de deuxième ligands est supérieur à 0,1, avantageusement supérieur à 0,5.

7. Procédé selon l'une des revendications précédentes, **caractérisé en ce que** le premier ligand monodentate répond à la formule générale (I) suivante : 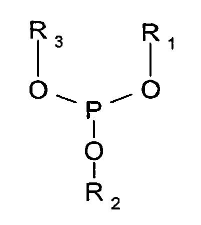 Dans laquelle
R₁, R₂, R₃ identiques ou différents représentent un radical alkyle linéaire ou ramifié ayant de 1 à 2 atomes de carbone pouvant comprendre des hétéroatomes, un radical aromatique ou cycloaliphatique substitué ou non pouvant comprendre des hétéroatomes, un radical aromatique ou cycloaliphatique substitué ou non pouvant comprendre des héréroatomes et
un ou plusieurs cycles sous forme condensée ou non, les radicaux R₁, R₂, R₃ pouvant être reliés entre eux deux à deux.

8. Procédé selon l'une des revendications précédentes, **caractérisé en ce que** le deuxième ligand répond à la formule générale (II) : 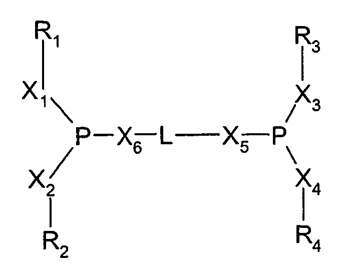 Dans laquelle,
R_{1'} R₂, R₃, R₄ identiques ou différent représentent un radical alkyle linéaire ou ramifié ayant de 1 à 12 atomes de carbone pouvant comprendre des hétéroatomes, un radical aromatiques ou cycloaliphatique substitué ou non pouvant comprendre des hétéroatomes et un ou plusieurs cycles sous forme condensée ou non, un radical alkylaryle, ou un radical arylalkyle, les radicaux R₁, et R₂, et/ou R₃ et R₄ pouvant être reliés entre eux
X₁, X₂, X₃, X₄, X₅, X₆ identiques ou différents représentent une liaison covalente, un atome d'oxygène ou un radical divalent - NR₅ - dans lequel R₅ représente un atome d'hydrogène ou un radical alkyle, aryle, sulfonyle cycloalkyle ou carbonylé, et
L représente une liaison covalente ou un radical divalent, alkyle linéaire ayant de 1 à 12 atomes de carbone pouvant comprendre des hétéroatomes, un radical divalent cycloaliphatique ou aromatique pouvant comprendre des hétéroatomes, substitué ou non, pouvant comprendre plusieurs cycles sous forme condensée ou non, un radical divalent alkylaryle, ou arylakyle.

9. Procédé selon l'une des revendications précédentes, **caractérisé en ce que** le premier ligand monodentate est choisi dans le groupe comprenant le triphénylphosphite, le tritolylphosphite, le trithymolphosphite, et le deuxième ligand bidentate est choisi dans le groupe comprenant les composés de formules suivantes :

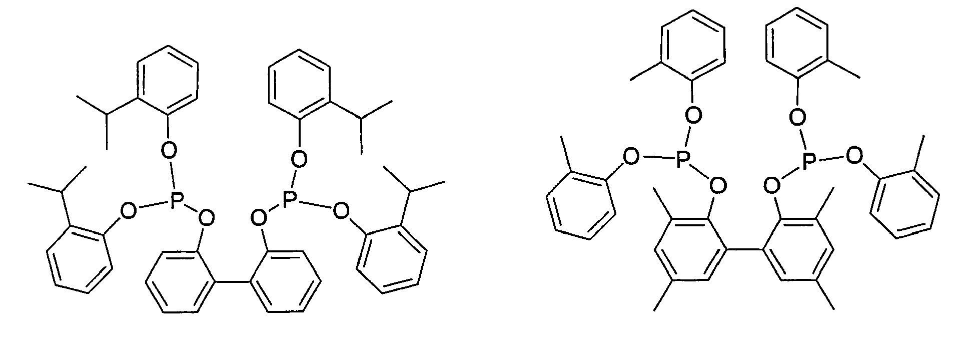

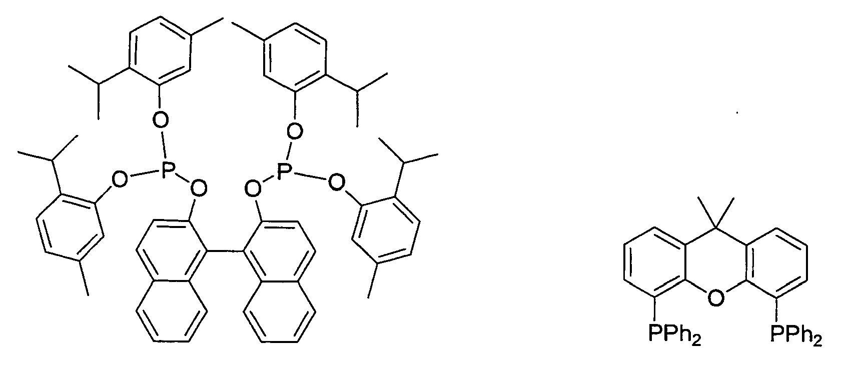

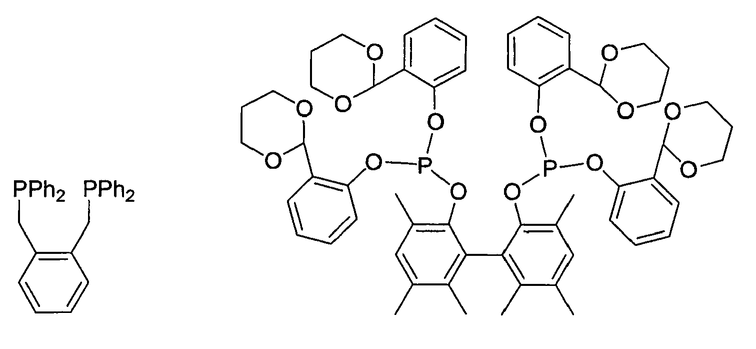

10. Procédé selon l'une des revendications précédentes, **caractérisé en ce que** la réaction est effectuée en milieu monophasique.

11. Procédé selon l'une des revendications précédentes **caractérisé en ce que** le catalyseur correspond à la formule générale (III):
M [L₁]x [L₂]y (III)
Dans laquelle
- M représente un métal de transition à activité catalytique défini ci-dessus.
- L₁ représente le premier ligand organophosphite monodentate
- L₂ représente le deuxième ligand organophosphoré pluridentate
x; y représentent un nombre décimal dans le système correspondant au nombre de mole du ligand respectif dans le système catalytique.

12. Procédé selon l'une des revendications précédentes, **caractérisé en ce que** l'élément métallique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure.

13. Procédé selon l'une des revendications précédentes, **caractérisé en ce que** le milieu réactionnel comprend un solvant du catalyseur miscible à la phase comprenant le composé à hydrocyaner à la température d'hydrocyanation.

14. Procédé selon l'une des revendications précédentes, **caractérisé en ce que** les composés des métaux de transition sont ceux du nickel et sont choisis dans le groupe comprenant :
les composés dans lesquels le nickel est au degré d'oxydation zéro comme le tétracyanonickelate de potassium K4 [(Ni(CN)4], le bis(acrylonitrile) nickel zéro, le bis(cyclooctadiène-1,5) nickel et les dérivés contenant des ligands comme le tétrakis(triphényl-phosphine) nickel zéro ;
les composés du nickel comme les carboxylates, carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

15. Procédé selon l'une des revendications précédentes, **caractérisé en ce que** les composés organiques comportant au moins une double liaison éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

16. Procédé selon l'une des revendications précédentes, **caractérisé en ce que** la quantité de composé du nickel ou d'un autre métal de transition utilisée est choisie de telle sorte qu'il y ait par mole de composé organique à hydrocyaner ou isomériser entre 10-4 et 1 mole de nickel ou de l'autre métal de transition mis en oeuvre et **en ce que** la quantité de composés de formule (I) ou formule (II) utilisée est choisie de telle sorte que le nombre de moles de ce composé rapporté à 1 mole de métal de transition soit de 0,5 à 100.

17. Procédé selon l'une des revendications précédentes, **caractérisé en ce que** la réaction d'hydrocyanation est réalisée à une température de 10°C à 200°C.

18. Procédé d'hydrocyanation en dinitriles de composés nitriles à insaturation éthylénique, par réaction avec le cyanure d'hydrogène, **caractérisé en ce que** l'on opère en présence d'un système catalytique selon l'une des revendications précédentes et un cocatalyseur consistant en au moins un acide de Lewis.

19. Procédé selon la revendication 18, **caractérisé en ce** les composés nitriles à insaturation éthylénique sont choisis parmi les nitriles aliphatiques à insaturation éthylénique comprenant les pentènenitriles linéaires comme le pentène-3-nitrile, le pentène-4-nitrile et leurs mélanges.

20. Procédé selon la revendication 19, **caractérisé en ce que** les pentènenitriles linéaires contiennent des quantités d'autres composés choisis dans le groupe comprenant le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène.

21. Procédé selon l'une des revendications 18 à 20, **caractérisé en ce que** l'acide de Lewis mis en oeuvre comme cocatalyseur est choisi parmi les composés des éléments des groupes Ib, IIb, IIIa, IIIb, IVa, IVb, Va, Vb, VIb, VIIb et VIII de la Classification périodique des éléments.

22. Procédé selon l'une des revendications 18 à 21, **caractérisé en ce que** l'acide de Lewis est choisi parmi les sels choisi dans le groupe des halogénures, sulfates, sulfonates, halogenoalkylsulfonates, perhalogénoalkylsulfonates, halogénoacétates, perhalogénoacétates, carboxylates et phosphates.

23. Procédé selon l'une des revendications 18 à 22, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium, le triphényl borane, l'isopropylate de titane et leurs mélanges .

24. Procédé selon l'une des revendications 18 à 23, **caractérisé en ce que** l'acide de Lewis mis en oeuvre représente de 0,01 à 50 moles par mole de composé de métal de transition.

25. Procédé **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur selon l'une des revendications 1 à 17.

26. Procédé selon la revendication 25, **caractérisé en ce que** le méthyl-2-butène-3-nitrile soumis à l'isomérisation est mis en oeuvre seul ou en mélange avec du méthyl-2-butène-2-nitrile, du pentène-4-nitrile, du pentène-3-nitrile, du pentène-2-nitrile, du butadiène, de l'adiponitrile, du méthyl-2-glutaroronitrile, de l'éthyl-2-succinonitrile ou du valéronitrile.

27. Procédé selon l'une des revendications 25 ou 26, **caractérisé en ce que** la réaction d'isomérisation est réalisée à une température de 10°C à 200°C.

## Claims

1. Process for the hydrocyanation of compounds comprising at least one ethylenic unsaturation by reaction with hydrogen cyanide in the presence of a catalytic system comprising a metal element possessing catalytic activity associated with organophosphorus ligands chosen from the group consisting of organophosphonites, organophosphinites, organophosphites, organophosphines and organophosphoramidites, **characterized in that** the catalytic system comprising at least one first ligand chosen from the group consisting of monodentate organophosphites and at least one second ligand chosen from the group consisting of polydentate organophosphorus ligands.

2. Process according to Claim 1, **characterized in that** the second organophosphorus ligand is a bidentate ligand.

3. Process according to Claim 1 or 2, **characterized in that** the second ligand is chosen from the group consisting of organophosphite, organophosphine, organophosphinite, organophosphonite and organophosphoramidite compounds.

4. Process according to one of the preceding claims, **characterized in that** the ratio of the number of second polydentate ligands, expressed as phosphorus atoms, to the number of atoms of metal element is greater than 0.5, preferably between 1 and 5.

5. Process according to one of the preceding claims, **characterized in that** the ratio of the total number of moles of first and second ligands, expressed as number of phosphorus atoms, to the number of atoms of metal element is between 1 and 100.

6. Process according to one of the preceding claims, **characterized in that** the ratio of the total number of moles of first ligand to the number of moles of second ligands is greater than 0.1, advantageously greater than 0.5.

7. Process according to one of the preceding claims, **characterized in that** the first monodentate ligand corresponds to the following general formula (I): 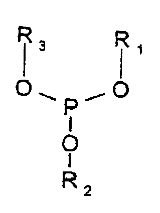 in which:
R₁, R₂ and R₃, which are identical or different, represent a linear or branched alkyl radical having from 1 to 2 carbon atoms which can comprise heteroatoms, a substituted or unsubstituted aromatic or cycloaliphatic radical which can comprise heteroatoms, or a substituted or unsubstituted aromatic or cycloaliphatic radical which can comprise heteroatoms and one or more rings in the fused or nonfused form, it being possible for the radicals R₁, R₂ and R₃ to be connected to one another in pairs.

8. Process according to one of the preceding claims, **characterized in that** the second ligand corresponds to the general formula (II): 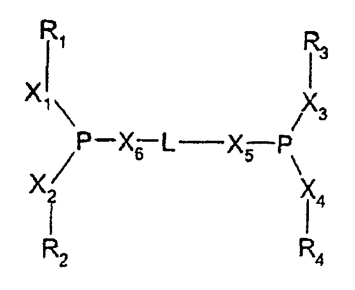 in which:
R₁, R₂, R₃ and R₄, which are identical or different, represent a linear or branched alkyl radical having from 1 to 12 carbon atoms which can comprise heteroatoms, a substituted or unsubstituted aromatic or cycloaliphatic radical which can comprise heteroatoms and one or more rings in the fused or nonfused form, an alkylaryl radical or an arylalkyl radical, it being possible for the radicals R₁ and R₂ and/or R₃ and R₄ to be connected to one another,
X₁, X₂, X₃, X₄, X₅ and X₆, which are identical or different, represent a covalent bond, an oxygen atom or a divalent radical -NR₅- in which R₅ represents a hydrogen atom or an alkyl, aryl, sulphonyl, cycloalkyl or carbonyl radical, and
L represents a covalent bond or a divalent linear alkyl radical having from 1 to 12 carbon atoms which can comprise heteroatoms, a substituted or unsubstituted divalent cycloaliphatic or aromatic radical which can comprise heteroatoms and which can comprise several rings in a fused or nonfused form, a divalent alkylaryl radical or a divalent arylalkyl radical.

9. Process according to one of the preceding claims, **characterized in that** the first monodentate ligand is chosen from the group consisting of triphenyl phosphite, tritolyl phosphite and trithymyl phosphite and the second bidentate ligand is chosen from the group consisting of the compounds with the following formulae:

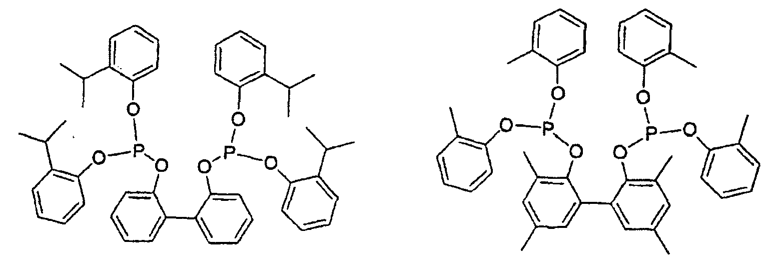

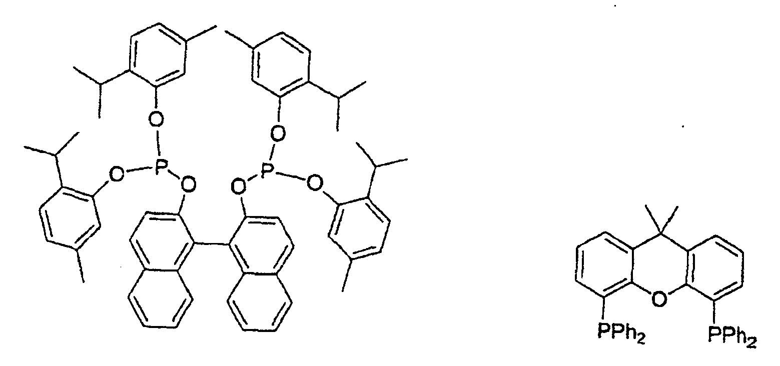

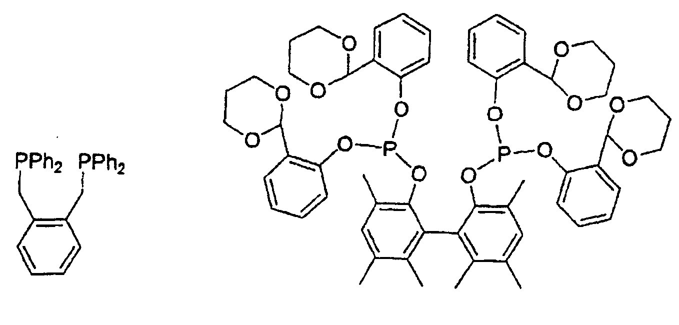

10. Process according to one of the preceding claims, **characterized in that** the reaction is carried out in a single-phase medium.

11. Process according to one of the preceding claims, **characterized in that** the catalyst corresponds to the general formula (III):
M[L₁]x[L₂]y (III)
in which:
- M represents a transition metal possessing catalytic activity defined below,
- L₁ represents the first monodentate organophosphite ligand,
- L₂ represents the second polydentate organophosphorus ligand,
- x and y represent a decimal number in the system corresponding to the number of moles of the respective ligand in the catalytic system.

12. Process according to one of the preceding claims, **characterized in that** the metal element is chosen from the group consisting of nickel, cobalt, iron, ruthenium, rhodium, palladium, osmium, iridium, platinum, copper, silver, gold, zinc, cadmium and mercury.

13. Process according to one of the preceding claims, **characterized in that** the reaction medium comprises a solvent for the catalyst which is miscible with the phase comprising the compound to be hydrocyanated at the hydrocyanation temperature.

14. Process according to one of the preceding claims, **characterized in that** the transition metal compounds are those of nickel and are chosen from the group consisting of:
compounds in which the nickel is in the zero oxidation state, such as potassium tetracyano-nickelate K₄[Ni(CN)₄], bis(acrylonitrile)nickel(0), bis(1,5-cyclooctadiene)nickel and derivatives comprising ligands, such as tetrakis(triphenylphosphine)nickel(0);
nickel compounds, such as carboxylates, carbonate, bicarbonate, borate, bromide, chloride, citrate, thiocyanate, cyanide, formate, hydroxide, hydrophosphite, phosphite, phosphate and derivatives, iodide, nitrate, sulphate, sulphite, arylsulphonates and alkylsulphonates.

15. Process according to one of the preceding claims, **characterized in that** the organic compounds comprising at least one ethylenic double bond are chosen from diolefins, such as butadiene, isoprene, 1,5-hexadiene or 1,5-cyclooctadiene, aliphatic nitriles possessing ethylenic unsaturation, particularly linear pentenenitriles, such as 3-pentenenitrile or 4-pentenenitrile, monoolefins, such as styrene, methylstyrene, vinylnaphthalene, cyclohexene or methylcyclohexene, and mixtures of several of these compounds.

16. Process according to one of the preceding claims, **characterized in that** the amount of nickel compound or of compound of another transition metal used is chosen so that between 10-4 and 1 mol of nickel or of the other transition metal are employed per mole of organic compound to be hydrocyanated or isomerized and **in that** the amount of compound of formula (I) or formula (II) used is chosen so that the number of moles of this compound with respect to 1 mol of transition metal is from 0.5 to 100.

17. Process according to one of the preceding claims, **characterized in that** the hydrocyanation reaction is carried at a temperature of 10 to 200°C.

18. Process for the hydrocyanation to give dinitriles of nitrile compounds possessing ethylenic unsaturation by reaction with hydrogen cyanide, **characterized in that** the reaction is carried out in the presence of a catalytic system according to one of the preceding claims and a cocatalyst comprising at least one Lewis acid.

19. Process according to Claim 18, **characterized in that** the nitrile compounds possessing ethylenic unsaturation are chosen from aliphatic nitriles possessing ethylenic unsaturation comprising linear pentenenitriles, such as 3-pentenenitrile, 4-pentenenitrile and their mixtures.

20. Process according to Claim 19, **characterized in that** the linear pentenenitriles comprise amounts of other compounds chosen from the group consisting of 2-methyl-3-butenenitrile, 2-methyl-2-butenenitrile, 2-pentenenitrile, valeronitrile, adiponitrile, 2-methylglutaronitrile, 2-ethylsuccinonitrile or butadiene.

21. Process according to one of Claims 18 to 20, **characterized in that** the Lewis acid employed as cocatalyst is chosen from compounds of elements from Groups Ib, IIb, IIIa, IIIb, IVa, IVb, Va, Vb, VIb, VIIb and VIII of the Periodic Table of the Elements.

22. Process according to one of Claims 18 to 21, **characterized in that** the Lewis acid is chosen from salts chosen from the group of the halides, sulphates, sulphonates, haloalkylsulphonates, perhaloalkylsulphonates, haloacetates, perhaloacetates, carboxylates and phosphates.

23. Process according to one of Claims 18 to 22, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulphate, stannous tartrate, indium chloride, indium trifluoromethylsulphonate, indium trifluoroacetate, chlorides or bromides of rare earth elements, such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride, triphenylborane, titanium isopropoxide, and their mixtures.

24. Process according to one of Claims 18 to 23, **characterized in that** the Lewis acid employed represents from 0.01 to 50 mol per mole of transition metal compound.

25. Process, **characterized in that** isomerization to give pentenenitriles of the 2-methyl-3-butenenitrile present in the reaction mixture originating from the hydrocyanation of butadiene is carried out in the absence of hydrogen cyanide, the operation being carried out in the presence of a catalyst according to one of Claims 1 to 17.

26. Process according Claims 25, **characterized in that** the 2-methyl-3-butenenitrile subjected to the isomerization is employed alone or as a mixture with 2-methyl-2-butenenitrile, 4-pentenenitrile, 3-pentenenitrile, 2-pentenenitrile, butadiene, adiponitrile, 2-methylglutaronitrile, 2-ethylsuccinonitrile or valeronitrile.

27. Process according to either of Claims 25 and 26, **characterized in that** the isomerization reaction is carried out with a temperature of 10°C to 200°C.

## Patentansprüche

1. Verfahren zur Hydrocyanierung von Verbindungen, die wenigstens eine ethylenische Ungesättigtheit umfassen, durch Reaktion mit Cyanwasserstoffsäure, in Anwesenheit eines katalytischen Systems, das ein Metallelement mit katalytischer Aktivität umfasst, verbunden mit Organophosphorliganden, ausgewählt aus der Gruppe, die die Organophosphonite, Organophosphinite, Organophosphite, Organophosphine und Organophosphoramidite umfasst, **dadurch gekennzeichnet, dass** das katalytische System wenigstens einen ersten Liganden umfasst, der aus der Gruppe der einzähligen Organophosphite ausgewählt ist, und wenigstens einen zweiten Liganden, der aus der Gruppe der mehrzähligen Organophosphorliganden ausgewählt ist.

2. Verfahren nach Anspruch 1, **dadurch gekennzeichnet, dass** der zweite Organophosphorligand ein zweizähliger Ligand ist.

3. Verfahren nach Anspruch 1 oder 2, **dadurch gekennzeichnet, dass** der zweite Ligand aus der Gruppe ausgewählt ist, die Organophosphitverbindungen, Organophosphinverbindungen, Organophosphinitverbindungen, Organophosphonitverbindungen, Organophoramiditverbindungen umfasst.

4. Verfahren nach einem der vorangehenden Ansprüche, **dadurch gekennzeichnet, dass** das Verhältnis der Anzahl der zweiten mehrzähligen Liganden, ausgedrückt in Phosphoratomen, zu der Anzahl der Metallelementatome größer als 0,5 ist und vorzugsweise zwischen einschließlich 1 und 5 liegt.

5. Verfahren nach einem der vorangehenden Ansprüche, **dadurch gekennzeichnet, dass** das Verhältnis zwischen der Gesamtanzahl der Mole des ersten und zweiten Liganden, ausgedrückt in der Anzahl der Phosphoratome, und der Anzahl der Metallelementatome zwischen einschließlich 1 und 100 liegt.

6. Verfahren nach einem der vorangehenden Ansprüche, **dadurch gekennzeichnet, dass** das Verhältnis zwischen der Gesamtzahl der Mole des ersten Liganden und Anzahl der Mole des zweiten Liganden größer als 0,1 ist, vorzugsweise größer als 0,5 ist.

7. Verfahren nach einem der vorangehenden Ansprüche, **dadurch gekennzeichnet, dass** der erste einzählige Ligand der folgenden allgemeinen Formel (I) entspricht: 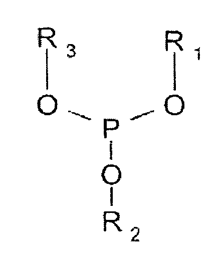 wobei
R₁, R₂, R₃ gleich oder verschieden sind und einen linearen oder verzweigten Alkylrest mit 1 bis 2 Kohlenstoffatomen, der Heteroatome enthalten kann, einen aromatischen oder cycloaliphatischenRest, der substituiert oder nicht substituiert ist und Heteroatome enthalten kann, einen aromatischen oder cycloaliphatischen Rest, der substituiert oder nicht substituiert ist und Heteroatome enthalten kann, und ein oder mehrere Ringe in kondensierter oder nichtkondensierter Form darstellen, wobei jeweils zwei der Reste R₁, R₂, R₃ untereinander verbunden sein können.

8. Verfahren nach einem der vorangehenden Ansprüche, **dadurch gekennzeichnet, dass** der zweite Ligand der allgemeinen Formel (II) entspricht: 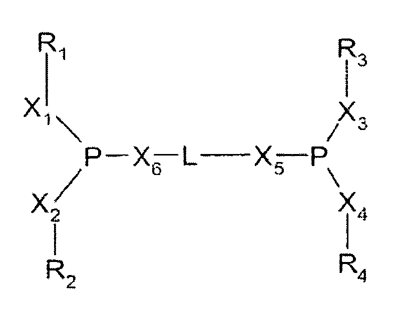 wobei
R₁, R₂, R₃, R₄ gleich oder verschieden sind und einen linearen oder verzweigten Alkylrest mit 1 bis 12 Kohlenstoffatomen, der Heteroatome enthalten kann, einen aromatischen oder cycloaliphatischenRest, der substituiert oder nicht substituiert ist und Heteroatome enthalten kann, und ein oder mehrere Ringe in kondensierter Form oder nichtkondensierter Form, ein Alkylarylrest oder einen Arylalkylrest darstellen, wobei die Reste R₁ und R₂ und/oder R₃ und R₄ untereinander verbunden sein können,
X₁, X₂, X₃, X₄, X₅, X₆, die gleich oder verschieden sind, eine kovalente Verbindung, ein Sauerstoffatom oder einen zweiwertigen Rest - NR₅ - darstellen, wobei R₅ ein Wasserstoffatom oder einen Alkyl-, Aryl-, Sulfonyl-, Cycloalkyl- oder Carbonylrest darstellt, und
L eine kovalente Bindung oder einen zweiwertigen linearen Alkylrest mit 1 bis 12 Kohlenstoffatomen, der Heteroatome enthalten kann, einen zweiwertigen cycloaliphatischen oder aromatischen Rest, der Heteroatome enthalten kann und substituiert oder nichtsubstituiert ist und mehrere Ringe in kondensierter oder nicht kondensierter Form enthalten kann, einen zweiwertigen Alkylaryl- oder Arylakylrest darstellt.

9. Verfahren nach einem der vorangehenden Ansprüche, **dadurch gekennzeichnet, dass** der erste einzählige Ligand aus der Gruppe ausgewählt ist, die Triphenylphosphit, Tritolylphosphit, Trithymolphosphit umfasst, und der zweite zweizählige Ligand aus der Gruppe ausgewählt ist, die die Verbindungen der folgenden Formeln umfasst:

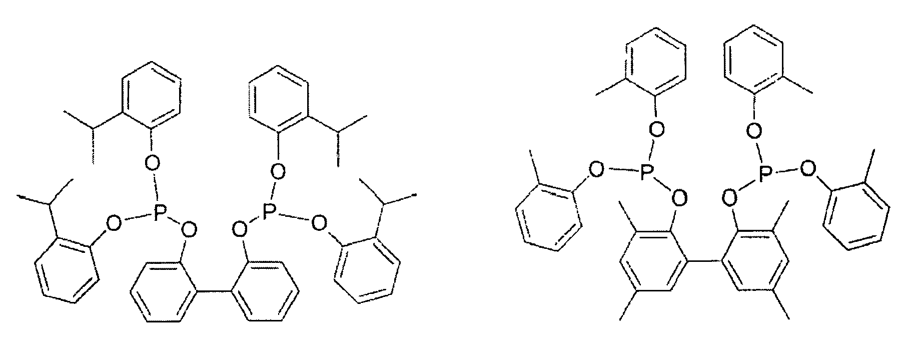

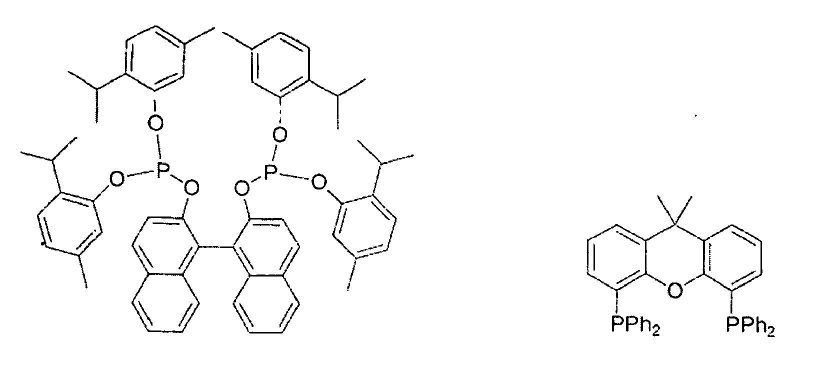

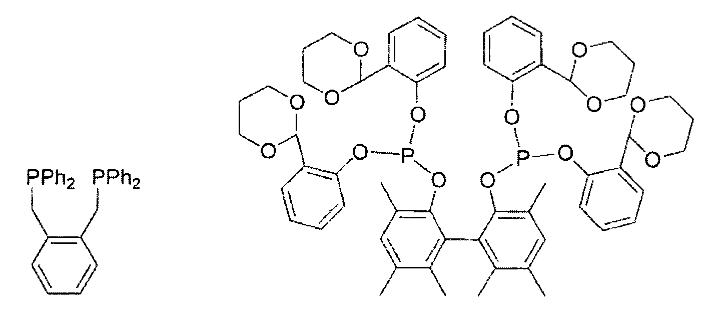

10. Verfahren nach einem der vorangehenden Ansprüche, **dadurch gekennzeichnet, dass** die Reaktion in einem einphasigen Medium durchgeführt wird.

11. Verfahren nach einem der vorangehenden Ansprüche, **dadurch gekennzeichnet, dass** der Katalysator der allgemeinen Formel (III) entspricht:
M [L₁]ₓ [L₂]_{y} (III)
wobei
- M ein Übergangsmetall mit oben definierter katalytischer Aktivität darstellt,
- L₁ den ersten einzähligen organischen Phosphorliganden darstellt,
- L₂ den zweiten mehrzähligen organischen Phosphorliganden darstellt,
x; y eine Dezimalzahl in dem System darstellen, die der Anzahl der Mole des jeweiligen Liganden im katalytischen System entspricht.

12. Verfahren nach einem der vorangehenden Ansprüche, **dadurch gekennzeichnet, dass** das Metallelement aus der Gruppe ausgewählt ist, die Nickel, Cobalt, Eisen, Ruthenium, Rhodium, Palladium, Osmium, Iridium, Platin, Kupfer, Silber, Gold, Zink, Cadmium, Quecksilber umfasst.

13. Verfahren nach einem der vorangehenden Ansprüche, **dadurch gekennzeichnet, dass** das Reaktionsmedium ein Katalysatorlösungsmittel umfasst, das mit der Phase mischbar ist, die die bei der Hydrocyanierungstemperatur zu hydrocyanierende Verbindung umfasst.

14. Verfahren nach einem der vorangehenden Ansprüche, **dadurch gekennzeichnet, dass** die Verbindungen der Übergangsmetalle Nickelverbindungen sind und aus der Gruppe ausgewählt sind, die umfasst: die Verbindungen, in denen Nickel den Oxidationsgrad null hat, wie Kaliumtetracyanonickelat K₄[(Ni(CN)₄], Bis-(acrylnitril)-nickel(null), Bis(1,5-cyclooctadien)nickel und die Derivate, die Liganden wie Tetrakis(triphenylphosphin)nickel(null) enthalten;
die Nickelverbindungen wie die Carboxylate, Carbonat, Hydrogencarbonat, Borat, Bromid, Chlorid, Citrat, Thiocyanat, Cyanat, Formiat, Hydroxid, Hydrophosphit, Phosphit, Phosphat und Derivate, Jodid, Nitrat, Sulfat, Sulfit, Aryl- und Alkylsulfonate.

15. Verfahren nach einem der vorangehenden Ansprüche, **dadurch gekennzeichnet, dass** die organischen Verbindungen, die wenigstens eine Ethylendoppelbindung enthalten, aus den Diolefinen wie Butadien, Isopren, 1,5-Hexadien, 1,5-Cyclooctadien, den aliphatischen Nitrilen mit Ethylenungesättigtheit, insbesondere den linearen Pentennitrilen wie 3-Pentennitril, 4-Pentennitril, den Monoolefinen wie Styrol, Methylstyrol, Vinylnaphthalin, Cyclohexen, Methylcyclohexen sowie den Gemischen mehrerer dieser Verbindungen ausgewählt sind.

16. Verfahren nach einem der vorangehenden Ansprüche, **dadurch gekennzeichnet, dass** die Menge der Verbindungen von Nickel oder von einem anderen verwendeten Übergangsmetall derart ausgewählt ist, dass sie pro Mol organische Verbindung, die zu hydrocyanieren oder isomerisieren ist, zwischen 10⁻⁴ und 1 Mol Nickel oder des anderen eingesetzten Übergangsmetalls liegt, und **dadurch**, dass die verwendete Menge der Verbindungen der Formel (I) oder Formel (II) derart ausgewählt ist, dass die Anzahl der Mole dieser Verbindung, bezogen auf 1 Mol Übergangsmetall, von 0,5 bis 100 beträgt.

17. Verfahren nach einem der vorangehenden Ansprüche, **dadurch gekennzeichnet, dass** die Hydrocyanierungsreaktion bei einer Temperatur von 10 °C bis 200 °C durchgeführt wird.

18. Verfahren zur Hydrocyanierung in Dinitrile von Nitrilverbindungen mit Ethylenungesättigtheit durch Reaktion mitCyanwasserstoffsäure, **dadurch gekennzeichnet, dass** in Anwesenheit eines katalytischen Systems nach einem der vorangehenden Ansprüche und eines Cokatalysators, der aus mindestens einer Lewis-Säure besteht, vorgegangen wird.

19. Verfahren nach Anspruch 18, **dadurch gekennzeichnet, dass** die Nitrilverbindungen mit Ethylenungesättigtheit aus den aliphatischen Nitrilen mit Ethylenungesättigtheit ausgewählt sind, die lineare Pentennitrile wie 3-Pentennitril, 4-Pentennitril und ihre Gemische umfassen.

20. Verfahren nach Anspruch 19, **dadurch gekennzeichnet, dass** die linearen Pentennitrile Mengen anderer Verbindungen enthalten, die aus der Gruppe ausgewählt sind, die 2-Methyl-3-butennitril, 2-Methyl-2-butennitril, 2-Pentennitril, Valeronitril, Adiponitril, 2-Methyl-glutaronitril, 2-Ethyl-succinonitril oder Butadien umfasst.

21. Verfahren nach einem der Ansprüche 18 bis 20, **dadurch gekennzeichnet, dass** die als Cokatalysator eingesetzte Lewis-Säure aus den Verbindungen der Elemente der Gruppen Ib, IIb, IIIa, IIIb, IVa, IVb, Va, Vb, VIb, VIIb und VIII des Periodensystems der Elemente ausgewählt ist.

22. Verfahren nach einem der Ansprüche 18 bis 21, **dadurch gekennzeichnet, dass** die Lewis-Säure aus den Salzen ausgewählt ist, ausgewählt aus der Gruppe der Halogenide, Sulfate, Sulfonate, Halogenalkylsulfonate, Perhalogenalkylsulfonate, Halogenacetate, Perhalogenacetate, Carboxylate und Phosphate.

23. Verfahren nach einem der Ansprüche 18 bis 22, **dadurch gekennzeichnet, dass** die Lewis-Säure aus Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumchlorid, Indium-trifluormethylsulfonat, Indiumtrifluoracetat, den Chloriden oder Bromiden der Elemente der Seltenen Erden wie Lanthan, Cerium, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid, Triphenylboran, Titanisopropylat und ihren Gemischen ausgewählt ist.

24. Verfahren nach einem der Ansprüche 18 bis 23, **dadurch gekennzeichnet, dass** die eingesetzte Lewis-Säure von 0,01 bis 50 Mol je Mol Übergangsmetallverbindung darstellt.

25. Verfahren, **dadurch gekennzeichnet**, das die Isomerisierung des im Reaktionsgemisch vorhandenen, aus der Hydrocyanierung des Butadiens stammenden 2-Methyl-3-butennitrils in Pentennitrile bei Abwesenheit von Cyanwasserstoffsäure durchgeführt wird, indem man in Anwesenheit eines Katalysators nach einem der Ansprüche 1 bis 17 vorgeht.

26. Verfahren nach Anspruch 25, **dadurch gekennzeichnet, dass** das der Isomerisierung unterzogene 2-Methyl-3-butennitril allein oder gemischt mit 2-Methyl-2-butennitril, 4-Pentennitril, 3-Pentennitril, 2-Pentennitril, Butadien, Adiponitril, 2-Methyl-glutaronitril, 2-Ethyl-succinonitril oder Valeronitril eingesetzt wird.

27. Verfahren nach einem der Ansprüche 25 oder 26, **dadurch gekennzeichnet, dass** die Isomerisierungsreaktion bei einer Temperatur von 10°C bis 200°C durchgeführt wird.
